# EUROPEAN PATENT SPECIFICATION

(11) **EP 2 214 653 B1**
(45) Date of publication and mention of the grant of the patent: **09.03.2016**
(21) Application number: 08846560.4
(22) Date of filing: 29.10.2008
(51) Int. Cl.: A61K 9/48, A61K 47/48, A61K 31/22, A61K 31/232, A61K 31/366, A61K 31/40, A61K 31/405, A61K 31/505

(54) **PHARMACEUTICAL COMPOSITIONS CONTAINING STATINS AND OMEGA-3 FATTY ACIDS DERIVATIVES AND THEIR SOLID FORMULATIONS FOR ORAL USE**
PHARMAZEUTISCHE ZUSAMMENSETZUNG MIT STATINEN UND OMEGA-3-FETTSÄUREDERIVATEN SOWIE FESTE FORMULIERUNGEN DARAUS ZUR ORALEN ANWENDUNG
COMPOSITIONS PHARMACEUTIQUES CONTENANT DES STATINES ET DES DÉRIVÉS D'ACIDES GRAS OMÉGA 3 ET LEURS FORMULATIONS SOLIDES POUR ADMINISTRATION ORALE

(30) Priority: 08.11.2007 IT MI20072142
(43) Date of publication of application: 11.08.2010
(73) Proprietor: SPA - Società Prodotti Antibiotici S.p.A., 20143 Milano (IT)
(72) Inventor: Zarmanian, Iervant, 1010 Wien (AT)
(74) Representative: Minoja, Fabrizio
(86) International application number: PCT/EP2008/009118
(87) International publication number: WO 2009/059717

(56) References cited:
- EP-A- 0 336 662
- EP-A- 0 470 452
- EP-A- 1 803 440
- WO-A-02/43659
- WO-A-95/16661
- WO-A-2006/096806
- WO-A-2007/103557
- WO-A-2008/146016
- US-A1- 2006 034 815
- US-B1- 6 234 464

## Description

This invention concerns pharmaceutical compositions comprising microcapsules or solid inclusion complexes of ω-3 polyunsaturated acids ethyl esters or suitable salts of the said acids, and competitive inhibitors of HMG-CoH reductase (statins) and their solid dosage forms for the treatment of subjects with hyperlipidemia or subjects with coronary heart disease, cardiac insufficiency or at risk of cardiovascular or cerebrovascular events, with results better than those that can be obtained by administration of either ω-3 polyunsaturated acids or statins alone.

### Base of the invention

Coronary heart disease is the most common cause of death in the western world and atherosclerosis is the most important etiopathogenetic factor of this pathology.

High blood cholesterol and lipid levels are atherosclerosis risk factors.

HMG-CoA reductase inhibitors (statins) are effective in reducing blood cholesterol levels, in particular low density lipoprotein cholesterol (LDL-C) (Grundy SM, Drug Treatment of Hyperlipidemia, Mercel Dekker Inc., New York, 1991, p. 139-167).

Examples of statins useful in the context of the present invention are as follows:
- Simvastatin, described in European patent no. 33538
- Pravastatin, described in European patent no. 33538
- Fluvastatin, described in patent WO8402131
- Atorvastatin, described in US patent 4,681,893 and its calcium salt, described in US patent 5,273,995
- Rosuvastatin, described in European patent 521,471

- High triglyceride levels too are nowadays considered an independent risk factor of atherosclerosis.

Several studies proved that the ω-3 polyunsaturated acids reduce both the triglyceride concentrations and, even at low doses, the level of postprandial lipids (Harris WS, J Lipid Res 1989 Jun 30 (6) : 785-807*;* Harris WS, Eur Heart J 2001, Suppl 3, Suppl D, D59).

Moreover, they increase HDL cholesterol levels and redistribute LDL sub-fractions by increasing the ratio between the larger and less atherogenous fractions and the smaller, denser and more atherogenous ones (Calabresi L. et al., Atheroscl 2000, 148 : 387*;* Nordoy A. et al., Nutr Metab cardiovasc Dis 2001, 11 : 7).

Ethyl esters of ω-3 polyunsaturated acids, in particular of eicosapentaenoic acid (EPA) and docosahexaenoic acid (DHA), can be obtained through known processes, such as those described in patents WO 89/11521, US 5130061, IT 1235879, EP 292846, US 4377526 and other patents.

The contents in EPA and DHA ethyl esters is generally higher than 25% and preferably not lower than 80-85%. In these mixtures the EPA to DHA ethyl ester ratio is generally between 0.9 and 1.5.

Gelatin coated microcapsules of EPA and DHA ethyl esters can be prepared through known processes and their dissolution in gastric environment can be modulated by glutaric aldehyde treatment.

In alternative, microcapsules masking better the taste and aftertaste of the polyunsaturated acids can be prepared by using gastroresistant cellulose derivatives as described in EP 0 336 662 A2 patent application.

Inclusion complexes of EPA and DHA ethyl esters with *β*-cyclodextrin can be obtained as described in patents EP 0 470 452 A2 and USP 5,189,149, incorporated herein. Salts of polyunsaturated ω-3 acids with amino alcohols or with lysine and arginine are described in patents USP 5,869,714 and USP 5,750,572 respectively.

WO2006/096806 A discloses unit dosages comprising a combination of statins with ω-3 fatty acids for the treatment of hyperlipidemia or for the prevention of cardiovascular events. In these formulations the ethyl ester or a triglyceride of ω-3 polyunsaturated acids is used as solvents for the statins.

Pharmaceutical solid dosage units comprising the combination of an ω-3 fatty acid derivative with a statin are also known from EP 1 803 440 A and from WO2007/103557 A.

Several large clinical trials demonstrated that the statins significantly reduced the incidence of cardiovascular disease and mortality in high blood cholesterol patients, during both primary and secondary prevention *(*Scandinavian Simvastatin Survival Study, Lancet 1994, 344 : 1383*;* LIPID Study Group, N Eng J Med 1998, 339 : 1349*;* Sheperd J et al., N Eng J Med 1995, 3331).

Moreover, several studies demonstrated that the statins, independently of their action on cholesterol levels, have favourable cardiovascular effects such as antioxidant and anti-inflammatory effects as well as effects on the endothelial function, on nitric oxide production, on macrophage proliferation, on platelets and on the coagulation/fibrinolysis system.

Epidemiologic and intervention studies demonstrated that the *ω*-3 fatty acids display cardio-protective properties, independent of their effects on plasma lipids. In the Gissi-Prevention study on more than 11,000 patients with a history of myocardial infarction already treated to the best of knowledge, additional treatment with 1 g daily of concentrated ethyl esters of *ω*-3 polyunsaturated acids was able to reduce total mortality by 20% and the incidence of sudden death by 45% (The Gissi Prevention Group, Lancet 1999; 354 : 447). Similar results were obtained in the DART study (Burr ML et al. Lancet 1989 Sep 30, 2 (8666) : 575-61*).* In several observational studies the intake of ω-3 acids and the blood concentrations of them were related to a reduced risk of sudden death (Hu FB et al, JAMA 2002, 287:1815-2*,* Albert et al, N Eng J Med 2002, 346 : 1113-8*,* Siskovich DS et al. JAMA 1995 : 274 : 1363-7*).*

Preclinical and clinical pharmacology studies were able to evidence various mechanisms responsible for their cardio-protective action; worth mentioning in particular, is an antiarrhythmic effect as well as a modification in the eicosaenoid picture, an increased nitric oxide production, a reduction in the adhesion molecules and inflammation mediators, and stabilization of the atheromatous plaque (Calder PC, Clinical Science 2004, 107 : 1).

Moreover, several studies evidenced the usefulness of joint administration of ω-3 polyunsaturated acids and statins.

In patients with various types of hyperlipidemia treated with statins, the additional therapy with ω-3, in particular with EPA and DHA ethyl esters, enhanced their normalizing action on lipids, in particular triglyceride reduction, HDL cholesterol increase, and postprandial blood fat reduction, as well as reduction of non HDL cholesterol and redistribution of LDL sub-fractions. These results were obtained by combining the ω-3 derivatives with statin (Durrington PN et al, Heart 2001, 85 : 544*;* Davidson MH et al, Am J Cardiol 1997, 80 : 797*;* Nordoy A et al, J Int Med 1998, 243 : 163*,* Nordoy A et al, Arterioscler Thromb Vasc Biol 2000, 20 : 259); pravastatin (Nakamura N et al, Int J Clin Lab Res 1999, 29 : 22*;* Contacos C et al, Arterioscl Thromb 1993, 13 : 1755*;* Contacos C et al, Atherosclerosis 1994, 109 : 25); fluvastatin *(*Singer P, Prostaglandins Leukotrienes Essential Fatty Acids 2005, 72 : 379*)* and atorvastatin (Nordoy A et al. Nutr Metab Cardiovasc Dis 2001, 11 : 7, Chan DC et al, Eur J Clin Invest 2002, 32 : 429*).*

The synergistic action of the combined treatment with statins and ω-3 acids was demonstrated also as regards cardiovascular protection, appeared independent of the blood fat normalizing effect.

In the already mentioned Gissi Prevention study, also in the patients who had previously received pravastatin, administration of ω-3 acids led to greater mortality reduction, in particular sudden death, thanks to their direct antiarrhythmic action.

In the ***JELIS*** Japanese study involving 18,000 patients under treatment with simvastatin or pravastatin, addition of EPA ethyl esters to the treatment led to an 18-19% reduction in major coronary events, reduction that was significant in the secondary prevention subgroup (Yokoyama et al, Lancet 2007, 369:1090*).*

All the foregoing data would suggest the opportunity of a combined treatment with statins and ω-3 polyunsaturated acid derivatives.

Both normalization of the lipid picture and primary or secondary cardiovascular prevention require long term treatments, for which maximum patient's compliance is necessary. To this end, availability is desirable of pharmaceutical forms combining supply of the active principles and palatability, that is, forms able to mask the unacceptable taste of the ω-3 acids and affording stability and compatibility of the ingredients.

### Summary of the invention

As it was found that the EPA and DHA derivatives can be used in combination with statins to obtain therapeutic results in the cardiovascular field better than those obtainable with either treatment alone, it was considered necessary to make available to the patient, formulations containing both active ingredients and endowed with palatability and stability

The present invention, meeting these requirements, concerns pharmaceutical combinations where EPA and DHA derivatives are present in the solid form as microcapsules, inclusion complexes or salts and their combinations with statins are formulated in dosage units as hard gelatin capsules or sachets for extemporary suspensions.

The compositions of the invention are pharmaceutical compositions in solid dosage units comprising either salts of ω-3 polyunsaturated acids with aminoacids, choline or aminoalcohols or microcapsules comprising ethyl esters of ω-3 polyunsaturated acids or inclusion complexes of ethyl esters of ω-3 polyunsaturated acids with cyclodextrins, a statin as a competitive inhibitor of HMG-CoA reductase, and pharmacologically acceptable excipients.

### Description of the invention

The present invention meets the demand for pharmaceutical compositions of ethyl esters of ω-3 polyunsaturated acids, in particular EPA and DHA, formulated such as to ensure stability and compatibility of the active ingredients and capable of obtaining maximum patient's compliance for a long term treatment.

It was in fact demonstrated that in both hyperlipidemia therapy and primary and secondary prevention of, even major, cardiovascular events, the combined treatment with ω-3 polyunsaturated acids and statins gave better results than those attainable with either treatment alone.

EPA and DHA ethyl esters are oily, with an unpleasant odour and presenting high peroxidation potential; some statins, such as simvastatin, are sensitive to an oxidizing environment.

We found that these problems could be solved by preparing solid formulations of this combination in solid dose units.

To obtain these formulations we found that microcapsules or inclusion complexes of EPA and DHA ethyl esters, being solid, could satisfactorily be used incorporated with the statins, in powder or previously granulated form.

Gelatin microcapsules can be used, eventually treated with glutaric aldehyde in the amount desired, to slow down gastric release and reduce the incidence of an unpleasant aftertaste.

Similar results can be obtained by using gastroresistant microcapsules, prepared with suitable cellulose derivatives, such as cellulose acetate phthalate or ethyl cellulose.

Satisfactory could prove the use of an inclusion complex of EPA and DHA ethyl esters with *β*-cyclodextrin, that can be obtained with molecular ratios EPA and DHA ethyl esters : *β*-cyclodextrin of 1: 1 to about 3 : 1.

In alternative EPA and DHA salts with glycine, choline and amino alcohols can be used.

The statins can be used in preparation of the final formulation without further treatment, following granulometric evaluation and eventual sieving. It could however be preferable to mix the statins with suitable diluents such as lactose, mannitol, polyvinylpyrrolidone, cellulose and possibly with binders such as starch, gelatin, carboxymethyl cellulose and lubricants such as magnesium stearate, calcium stearate, polyethylene glycole then treat the mixtures with wet or dry granulation; through suitable sieving the granules will then be reduced to granulometries not dissimilar from those of the microcapsules or complexes or salts of the ω-3 acids.

Suitable pharmaceutical forms for administration of these compositions can prove the hard gelatin capsules.

In this case it will be advisable to add excipients such as aluminium stearate or magnesium or talc, to ensure gliding of the mixture and easier filling of the capsules.

Pharmaceutical forms more suitable in case the dosage unit contained higher doses of active ingredient, are the sachets for preparation of extemporary suspensions. In this case suitable diluents can be added such as silica or microcrystalline cellulose or lactose, surface active substances such as polysorbates or sodium lauryl sulphate to help suspension and, furthermore, flavouring agents or sweeteners.

In any case, further protection of the ω-3 acid derivatives or statins subject to oxidation, e.g., simvastatin, can be achieved through addition of antioxidants such as tocopherol, butylhydroxy anisole, or butylhydroxy toluol. When necessary acidifiers such as citric acid or ascorbic acid can be added.

The dose of ω-3 polyunsaturated acids, preferably EPA and DHA and its esters or salts to be included in the formulations, varies between 0.5 g and 4 g. Preferably, in accordance with this invention, the dose unit contains from 0.5 g to 2 g of ω-3 acids or their derivatives.

The quantity of statins to be enclosed in the dosage unit are as follows:

| | |
|---|---|
| Simvastatin | 10 - 20 mg |
| Pravastatin (as sodium salt) | 10 - 40 mg |
| Fluvastatin (as sodium salt) | 20 - 40 mg |
| Atorvastatin (as calcium salt) | 5 - 20 mg |
| Rosuvastatin (as calcium salt) | 5 - 20 mg |

The following examples illustrate the invention but do not intend limiting it whatsoever.

### Example no. 1

### Formulation in hard gelatin capsules

**Gelatin microcapsules of 85% EPA and DHA ethyl esters**

| | |
|---|---|
| equivalent to active ingredients | 1000 mg |
| Simvastatin | 10 mg |
| Precipitated silica | 50 mg |
| Magnesium stearate | 15 mg |
| DL-*α* tocopherol | 0.3 mg |

### Example no. 2

### Formulation in hard gelatin capsules

**Microcapsules of cellulose acetate phthalate of 85% EPA and DHA ethyl esters**

| | |
|---|---|
| equivalent to active ingredients | 500 mg |
| Atorvastatin (as trihydrate calcium salt) | 5 mg |
| Microcrystalline cellulose | 60 mg |
| Calcium stearate | 15 mg |
| Butylhydroxyanisole | 0.02 mg |

### Example no. 3

### Formulation in hard gelatin capsules

**Inclusion complex of EPA and DHA ethyl esters with β-cyclodextrin**

| | |
|---|---|
| equivalent_to active ingredients | 750 mg |
| Simvastatin | 10 mg |
| Microcrystalline cellulose | 20 mg |
| Butylhydroxyanisole | 0.04 mg |
| Ascorbic acid | 2 mg |
| Sodium lauryl sulphate | 3 mg |

### Example no. 4

### Formulation in hard gelatin capsules

**Gelatin microcapsules of 85% EPA and DHA ethyl esters**

| | |
|---|---|
| equivalent to active ingredients | 1000 mg |
| Pravastatin sodium salt | 20 mg |
| Precipitated silica | 50 mg |
| Magnesium stearate | 10 mg |
| DL-*α*-tocopherol | 0.3 mg |

### Example no. 5

### Formulation in hard gelatin capsules

**Gelatin microcapsules of 85% EPA and DHA ethyl esters**

| | |
|---|---|
| equivalent to active ingredients | 500 mg |
| Rosuvastatin (as calcium salt) | 5 mg |
| Microcrystalline cellulose | 10 mg |
| Magnesium stearate | 15 mg |

### Example no. 6

### Formulation in sachets for extemporary preparation of water suspensions

**Gelatin microcapsules of 85% EPA and DHA ethyl esters**

| | |
|---|---|
| equivalent to active ingredients | 2000 mg |
| Simvastatin | 20 mg |
| Microcrystalline cellulose | 200 mg |
| Polysorbate 80 | 12 mg |
| Sorbitol | 1000 mg |
| Orange flavouring | 80 mg |
| Raspberry flavouring | 50 mg |
| Butylhydroxyanisole | 0.5 mg |
| Citric acid | 2 mg |
| Vanillin | 15 mg |

### Example no. 7

### Formulation in sachets for extemporary preparation of water suspensions

**Inclusion complex of 85% EPA and DHA ethyl esters with β-cyclodextrin**

| | |
|---|---|
| equivalent to active ingredients | 2000 mg |
| Pravastatin sodium salt | 40 mg |
| Sorbitol | 1500 mg |
| Polysorbate 80 | 15 mg |
| Butylhydroxyanisole | 0.5 mg |
| Orange flavouring | 90 mg |
| Sodium saccharinate | 12 mg |

### Example no. 8

### Formulation in sachets for extemporary preparation of water suspensions

**Gelatin microcapsules of 85% EPA and DHA ethyl esters**

| | |
|---|---|
| equivalent to active ingredients | 1500 mg |
| Fluvastatin sodium salt equivalent to fluvastatin | 20 mg |
| Sorbitol | 1000 mg |
| Precipitated silica | 50 mg |
| Butylhydroxyanisole | 0.02 mg |
| Lemon flavouring | 100 mg |

### Example no. 9

**Formulation in sachets for extemporary preparation of water suspensions**

| | |
|---|---|
| EPA choline salt equivalent to EPA | 450 mg |
| DHA choline salt equivalent to DHA | 300 mg |
| Simvastatin | 20 mg |
| Cetomacrogol | 1000 mg |
| Maltodextrin | 40 mg |
| Saccharose | 1500 mg |
| Orange flavouring | 40 mg |
| Butylhydroxyanisole | 0.04 mg |
| Citric acid 2 mg | |

## Claims

1. Pharmaceutical compositions in solid dosage units comprising either salts of ω-3 polyunsaturated acids with aminoacids, choline or aminoalcohols or microcapsules comprising ethyl esters of ω-3 polyunsaturated acids or inclusion complexes of ethyl esters of ω-3 polyunsaturated acids with cyclodextrins, a statin as a competitive inhibitor of HMG-CoA reductase, and pharmacologically acceptable excipients.

2. Pharmaceutical compositions according to claim 1 wherein the ethyl esters of ω-3 polyunsaturated acids contain EPA and DHA esters in concentrations comprised between 80 and 90%.

3. Pharmaceutical compositions according to claim 1 wherein the ethyl esters of ω-3 polyunsaturated acids are coated with gelatin to form microcapsules.

4. Pharmaceutical compositions according to claim 1 wherein the ethyl esters of ω-3 polyunsaturated acids are in microcapsules of gastroresistant cellulose derivatives.

5. Pharmaceutical compositions according to claim 1 wherein the ethyl esters of ω-3 polyunsaturated acids are used as inclusion complexes with cyclodextrins.

6. Pharmaceutical compositions according to claim 1 comprising salts of ω-3 polyunsaturated acids with amino acids.

7. Pharmaceutical compositions according to claim 1 comprising salts of ω-3 polyunsaturated acids with lysine or glycine.

8. Pharmaceutical compositions according to claim 1 comprising salts of ω-3 polyunsaturated acids with choline.

9. Pharmaceutical compositions according to claim 1 comprising salts of ω-3 polyunsaturated acids aminoalcohols.

10. Pharmaceutical compositions according to claim 1 comprising simvastatin.

11. Pharmaceutical compositions according to claim 1 comprising a pravastain salt, preferably sodium salt.

12. Pharmaceutical compositions according to claim 1 comprising an atorvastatin salt, preferably calcium salt.

13. Pharmaceutical compositions according to claim 1 comprising a fluvastatin salt, preferably sodium salt.

14. Pharmaceutical compositions according to claim 1 comprising a rosuvastatin salt, preferably calcium salt.

15. Pharmaceutical compositions according to claim 1 wherein the excipients comprise ascorbic acid, tocopherol, butylhydroxytoluene, butylhydroxyanisole or other antioxidants.

16. Pharmaceutical compositions according to claims 1-15 wherein the statins are incorporated with suitable excipients in granules having suitable granulometry.

17. Dose units of the pharmaceutical compositions according to claims 1-16 in hard gelatin capsules.

18. Dose units wherein the pharmaceutical compositions according to claims 1-16 are blended with suitable flavouring agents and sweeteners and packaged in sachets.

19. Compositions as claimed in claims 1-18 for use in the treatment of hyperlipidemia and primary and secondary prevention of major cardiovascular events.

## Patentansprüche

1. Pharmazeutische Zusammensetzungen in festen Dosierungseinheiten, umfassend entweder Salze von mehrfach ungesättigten ω-3-Säuren mit Aminosäuren, Cholin oder Aminoalkoholen oder Mikrokapseln, die Ethylester von mehrfach ungesättigten ω-3-Säuren umfassen, oder Einschlusskomplexe von Ethylestern von mehrfach ungesättigten ω-3-Säuren mit Cyclodextrinen, ein Statin als kompetitiven Inhibitor der HMG-CoA-Reductase sowie pharmakologisch annehmbare Arzneimittelhilfsstoffe.

2. Pharmazeutische Zusammensetzungen gemäß Anspruch 1, wobei die Ethylester von mehrfach ungesättigten ω-3-Säuren EPA- und DHA-Ester in Konzentrationen zwischen 80 und 90% enthalten.

3. Pharmazeutische Zusammensetzungen gemäß Anspruch 1, wobei die Ethylester von mehrfach ungesättigten ω-3-Säuren mit Gelatine überzogen sind, wobei Mikrokapseln entstehen.

4. Pharmazeutische Zusammensetzungen gemäß Anspruch 1, wobei die Ethylester von mehrfach ungesättigten ω-3-Säuren in Mikrokapseln aus magensäureresistenten Cellulosederivaten vorliegen.

5. Pharmazeutische Zusammensetzungen gemäß Anspruch 1, wobei die Ethylester von mehrfach ungesättigten ω-3-Säuren als Einschlusskomplexe mit Cyclodextrinen verwendet werden.

6. Pharmazeutische Zusammensetzungen gemäß Anspruch 1, die Salze von mehrfach ungesättigten ω-3-Säuren mit Aminosäuren umfassen.

7. Pharmazeutische Zusammensetzungen gemäß Anspruch 1, die Salze von mehrfach ungesättigten ω-3-Säuren mit Lysin oder Glycin umfassen.

8. Pharmazeutische Zusammensetzungen gemäß Anspruch 1, die Salze von mehrfach ungesättigten ω-3-Säuren mit Cholin umfassen.

9. Pharmazeutische Zusammensetzungen gemäß Anspruch 1, die Salze von mehrfach ungesättigten ω-3-Säuren mit Aminoalkoholen umfassen.

10. Pharmazeutische Zusammensetzungen gemäß Anspruch 1, die Simvastatin umfassen.

11. Pharmazeutische Zusammensetzungen gemäß Anspruch 1, die ein Pravastatinsalz, vorzugsweise Natriumsalz, umfassen.

12. Pharmazeutische Zusammensetzungen gemäß Anspruch 1, die ein Atorvastatinsalz, vorzugsweise Calciumsalz, umfassen.

13. Pharmazeutische Zusammensetzungen gemäß Anspruch 1, die ein Fluvastatinsalz, vorzugsweise Natriumsalz, umfassen.

14. Pharmazeutische Zusammensetzungen gemäß Anspruch 1, die ein Rosuvastatinsalz, vorzugsweise Calciumsalz, umfassen.

15. Pharmazeutische Zusammensetzungen gemäß Anspruch 1, wobei die Arzneimittelhilfsstoffe Ascorbinsäure, Tocopherol, Butylhydroxytoluol, Butylhydroxyanisol oder andere Antioxidantien umfassen.

16. Pharmazeutische Zusammensetzungen gemäß Anspruch 1 bis 15, wobei die Statine mit geeigneten Arzneimittelhilfsstoffen in Granulate mit einer geeigneten Korngrößenverteilung eingearbeitet sind.

17. Dosierungseinheiten der pharmazeutischen Zusammensetzungen gemäß Anspruch 1 bis 16 in Hartgelatinekapseln.

18. Dosierungseinheiten, bei denen die pharmazeutischen Zusammensetzungen gemäß Anspruch 1 bis 16 mit geeigneten Aromastoffen und Süßungsmitteln gemischt und in Beutel verpackt sind.

19. Zusammensetzungen gemäß Anspruch 1 bis 18 zur Verwendung bei der Behandlung von Hyperlipidämie und der primären und sekundären Prävention von schwerwiegenden kardiovaskulären Ereignissen.

## Revendications

1. Compositions pharmaceutiques sous la forme d'unités posologiques solides comprenant, soit des sels d'acides polyinsaturés ω3 avec des acides aminés, de la choline ou des aminoalcools, soit des microcapsules comprenant des esters éthyliques d'acides polyinsaturés ω3 ou des complexes d'inclusion d'esters éthyliques d'acides polyinsaturés ω3 avec des cyclodextrines, une statine à titre d'inhibiteur compétitif de la HMG-CoA réductase, et des excipients pharmacologiquement acceptables.

2. Compositions pharmaceutiques selon la revendication 1, dans lesquelles les esters éthyliques d'acides polyinsaturés ω3 contiennent des esters EPA et DHA dans des concentrations comprises entre 80 et 90 %.

3. Compositions pharmaceutiques selon la revendication 1, dans lesquelles les esters éthyliques d'acides polyinsaturés ω3 sont enrobés de gélatine pour obtenir des microcapsules.

4. Compositions pharmaceutiques selon la revendication 1, dans lesquelles les esters éthyliques d'acides polyinsaturés ω3 sont présents dans des microcapsules de dérivés gastrorésistants de la cellulose.

5. Compositions pharmaceutiques selon la revendication 1, dans lesquelles les esters éthyliques d'acides polyinsaturés ω3 sont utilisés sous la forme de complexes d'inclusion avec des cyclodextrines.

6. Compositions pharmaceutiques selon la revendication 1, comprenant des sels d'acides polyinsaturés ω3 avec des acides aminés.

7. Compositions pharmaceutiques selon la revendication 1, comprenant des sels d'acides polyinsaturés ω3 avec de la lysine ou de la glycine.

8. Compositions pharmaceutiques selon la revendication 1, comprenant des sels d'acides polyinsaturés ω3 avec de la choline.

9. Compositions pharmaceutiques selon la revendication 1, comprenant des sels d'acides polyinsaturés ω3 avec des aminoalcools.

10. Compositions pharmaceutiques selon la revendication 1, comprenant de la simvastatine.

11. Compositions pharmaceutiques selon la revendication 1, comprenant un sel de pravastatine, de préférence le sel de sodium.

12. Compositions pharmaceutiques selon la revendication 1, comprenant un sel d'atorvastatine, de préférence le sel de calcium.

13. Compositions pharmaceutiques selon la revendication 1, comprenant un sel de fluvastatine, de préférence le sel de sodium.

14. Compositions pharmaceutiques selon la revendication 1, comprenant un sel de rosuvastatine, de préférence le sel de calcium.

15. Compositions pharmaceutiques selon la revendication 1, dans lesquelles les excipients comprennent de l'acide ascorbique, du tocophérol, du butylhydroxytoluène, du butylhydroxyanisole ou d'autres antioxydants.

16. Compositions pharmaceutiques selon les revendications 1 à 15, dans lesquelles les statines sont incorporées avec des excipients appropriés dans des granulés possédant une granulométrie appropriée.

17. Unités posologiques des compositions pharmaceutiques selon les revendications 1 à 16, dans des capsules de gélatine dure.

18. Unités posologiques dans lesquelles les compositions pharmaceutiques selon les revendications 1 à 16 sont mélangées avec des aromatisants et des édulcorants appropriés et sont conditionnées dans des sachets.

19. Compositions pharmaceutiques selon les revendications 1 à 18, pour leur utilisation dans le traitement de l'hyperlipidémie et la prévention primaire et secondaire d'événements cardio-vasculaires majeurs.
